# EUROPEAN PATENT APPLICATION

(11) **EP 4 357 360 A1**
(43) Date of publication of application: **24.04.2024**
(21) Application number: 22825190.6
(22) Date of filing: 30.05.2022
(51) Int. Cl.: C07K 16/08, A61P 31/12, G01N 33/576, A61K 39/00

(54) **HUMAN ANTIBODY SPECIFICALLY BINDING TO BINDING SITE OF HEPATOCYTE RECEPTOR OF HEPATITIS B VIRUS PRES1 ANTIGEN, AND USE THEREOF**

(30) Priority: 15.06.2021 KR 20210077615; 16.07.2021 KR 20210093625
(71) Applicant: ApitBio, Inc., Seoul 05855 (KR)
(72) Inventor: HONG, Hyo Jeong, Chuncheon-si, Gangwon-do 24316 (KR); JO, Kyunghee, Cheorwon-gun, Gangwon-do 24035 (KR); HONG, Jisu, Incheon 21377 (KR); CHOI, Young Jin, Seoul 04940 (KR); LEE, Jiwoo, Gwangju-si, Gyeonggi-do 12713 (KR)
(74) Representative: Cabinet Beau de Loménie
(86) International application number: PCT/KR2022/007679
(87) International publication number: WO 2022/265264

(57) **Abstract**

The present invention relates to an antibody that specifically binds to a receptor binding motif of a hepatitis B virus preS1 antigen, and uses of the same.

The antibody of the present invention targets diverse genotypes of HBV, and can be thus usefully used in fields that require neutralization of HBV or inhibition and treatment of hepatitis B virus infection.

## Description

### [Technical Field]

The present invention relates to a human antibody that specifically binds to a hepatocyte receptor binding motif of a hepatitis B virus preS1 antigen, and uses of the same.

### [Background Art]

Hepatitis B virus (HBV) infection is the most serious and prevalent problem in public health. The global prevalence of chronic HBV infection in 2016 is approximately 290 million people.

To date, a total of 10 distinct HBV genotypes (A to J) have been identified. Genotypes A and D are prevalent mainly in Africa and Europe, genotypes B and C are mainly prevalent in Asia, and genotypes E to J frequently emerge in Europe, America, and Asia. The most prevalent genotypes are A to D, accounting for approximately 90% of hepatitis B cases.

The HBV envelope contains three structurally related proteins, S (small), M (middle), and L (large) proteins. The S protein is the most abundant protein among these coat proteins, the M protein contains the preS2 and S domains, and the L protein contains the preS1, preS2, and S domains.

HBV-infected hepatocytes produce infectious HBV particles (virions) as well as non-infectious spherical and filamentous subviral particles. These subviral particles consist mainly of the S protein, and are generally produced in numbers as many as 1,000 to 10,000 times that of virions. These subviral particles are suspected to reduce the virus-specific immune response.

Unlike the S protein, preS1 of the L protein is mainly present in infectious virions. In addition, amino acids 20 to 26 (for genotypes A to C and F; 9 to 15 for genotype D; and 19 to 25 for genotype E) of preS1 mediate HBV infection by binding to NTCP (sodium taurocholate cotransporting polypeptide), a hepatocyte receptor. This receptor binding motif is highly conserved among HBV genotypes, and can thus be a target for the treatment and prevention of HBV infection.

Hepatitis B immune globulins (HBIGs) are used for preventive medical treatment after accidental or perinatal exposure to HBV. HBIGs are produced by collecting serum with high anti-S protein antibody titers. However, considering that the preS1 region is important for HBV infection, a preS1-specific monoclonal antibody (mAb) that can inhibit HBV infection may be a promising method for preventing and treating HBV infection. Clinically, the presence of anti-preS1 antibodies has been revealed to be associated with recovery in patients with hepatitis B. Recently, Li *et al.* have demonstrated that a human monoclonal antibody (2H5-A14) that specifically binds to amino acids 31 to 38 (genotypes A to C and F), which are the downstream portion of the hepatocyte receptor binding motif (amino acids 20 to 26, genotypes A to C and F) of preS1, not only prevents HBV infection in a mouse model transplanted with human hepatocytes, but also exhibits a therapeutic effect through Fc-mediated effector function (Dan Li, Wenhui He, Ximing Liu, Sanduo Zheng, Yonghe Qi, Huiyu Li, Fengfeng Mao, Juan Liu, Yinyan Sun, Lijing Pan, Kaixin Du, Keqiong Ye, Wenhui Li, Jianhua Sui. 2017. A potent human neutralizing antibody Fc-dependently reduces established HBV infections. eLife 6:e26738. DOI: https://doi.org/10.7554/eLife.26738).

A number of anti-preS1 mAbs with HBV-neutralizing activity have been produced from mice immunized with HBV particles or recombinant preS1 antigen (Heermann K, Goldmann U, Schwartz W, Seyffarth T, Baumgarten H, Gerlich W. 1984. Large surface proteins of hepatitis B virus containing the pre-s sequence. Journal of virology. 52:396-402; Pizarro JC, Vulliez-le Normand B, Riottot M-M, Budkowska A, Bentley GA. 2001. Structural and functional characterization of a monoclonal antibody specific for the preS1 region of hepatitis B virus. FEBS letters. 509:463-468; Kim JH, Gripon P, Bouezzedine F, Jeong MS, Chi SW, Ryu SE, Hong HJ. 2015. Enhanced humanization and affinity maturation of neutralizing anti-hepatitis B virus preS1 antibody based on antigen-antibody complex structure. FEBS letters. 589:193-200; Wi J, Jeong MS, Hong HJ. 2017. Construction and characterization of an anti-hepatitis B virus preS1 humanized antibody that binds to the essential receptor binding site. Journal of microbiology and biotechnology 27, 1336-1344; and the like). However, most antibodies produced in this way have a problem in that these cannot neutralize all HBV genotypes.

### [Disclosure]

### [Technical Problem]

Against this background, for more efficient prevention or treatment, the present inventors have made diligent efforts to provide a monoclonal antibody that can bind to diverse genotypes of HBV and which is specific to the hepatocyte receptor binding motif of preS1, as a result, they developed a human monoclonal antibody that binds to all genotypes A to F, which account for most of the hepatitis B virus, and which exhibits HBV infection inhibiting efficacy by binding to the hepatocyte receptor binding motif, thereby completing the present invention.

### [Technical Solution]

An object of the present invention is to provide a novel antibody that specifically binds to a receptor binding motif of a hepatitis B virus preS1 antigen.

Another object of the present invention is to provide a method for producing the antibody.

Still another object of the present invention is to provide a polynucleotide encoding the antibody, an expression vector containing the polynucleotide, and a host cell into which the vector is introduced.

Still another object of the present invention is to provide a pharmaceutical composition for prevention or treatment of HBV infection, containing the antibody.

Still another object of the present invention is to provide a method for preventing or treating HBV infection using the antibody.

Still another object of the present invention is to provide a method of providing information for diagnosis of HBV infection, which includes detecting preS1 present in a biological sample isolated from a subject suspected of being infected with HBV through an antigen-antibody reaction using the antibody.

Still another object of the present invention is to provide a composition for detection of HBV, containing the antibody.

Still another object of the present invention is to provide a kit for detection of HBV, containing the antibody.

### [Advantageous Effects]

The antibody of the present invention targets the sequences shared by diverse genotypes of HBV, can bind to diverse genotypes of HBV and exhibits excellent ability to bind to HBV, and can be thus usefully used not only in the detection and diagnosis of HBV infection, but also in fields requiring treatment.

### [Brief Description of Drawings]

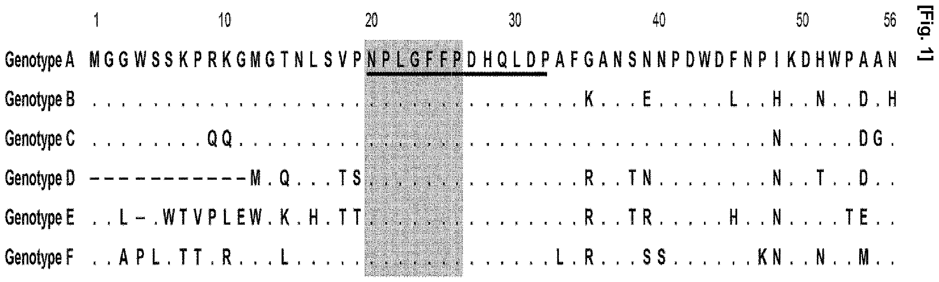

FIG. 1 illustrates the alignments of HBV preS1 N-terminal amino acid sequences (genotypes A to F, numbered according to HBV genotype A), in which amino acids 20 to 26 are essential receptor binding motifs of preS1 and are indicated in gray shade, and the sequence contained in Bio-preS1-L peptide is underlined;

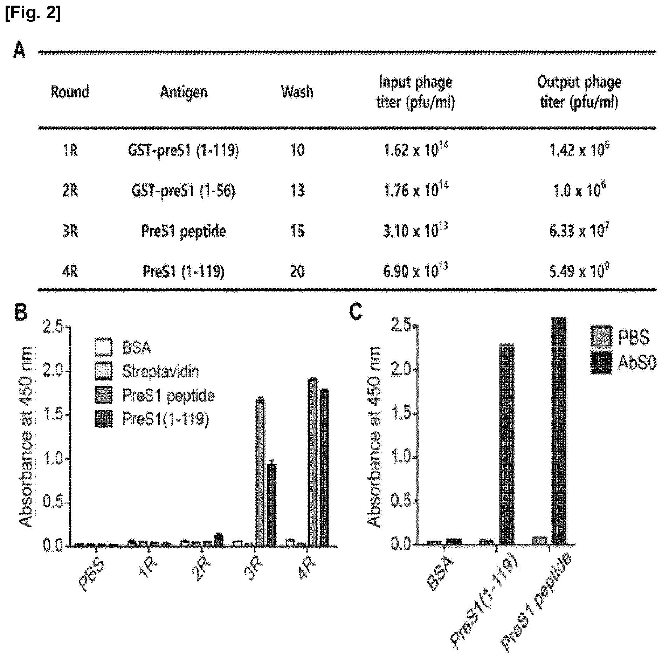

FIG. 2 illustrates the results of an experiment for isolating anti-preS1 monoclonal Fab from a human synthetic Fab library, FIG. 2A illustrates the input and output titers of phages for each round of panning, where pfu stands for plaque-forming unit, FIG. 2B illustrates positive polyclonal phage amplification determined by indirect ELISA, in which the fourth round of panning with the highest amplification against preS1 has been selected for the isolation of monoclonal Fab, and FIG. 2C illustrates the indirect ELISA results of AbS0 Fab phage, which has the best antigen binding activity to bio-preS1-L peptide among the positive Fab clones selected from the output of the fourth round of panning;

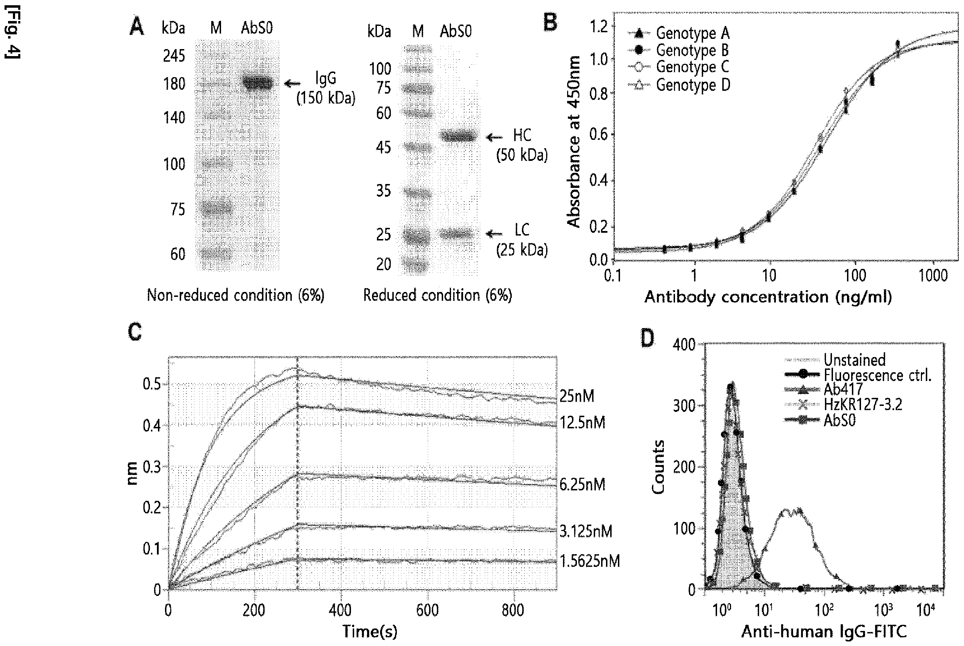

FIG. 3 illustrates analysis of the antigen binding activity of AbS0 mAb converted from AbS0 Fab to IgG1 and purified, FIG. 3A illustrates SDS-PAGE of AbS0 purified under non-reducing (NR, 6%) and reducing (R, 10%) conditions, where M denotes protein molecular weight markers, HC denotes a heavy chain, and LC denotes a light chain, FIG. 3B illustrates the antigen binding activity of purified AbS0 mAb to preS1 of HBV genotypes (A to D) evaluated by ELISA, FIG. 3C illustrates the results of affinity determination of AbS0 by BLI using Octet RED384, in which recombinant preS1 of HBV genotype A has been prepared by 2-fold serial dilution (25 nM, 12.5 nM, 6.25 nM, 3.125 nM, and 1.5625 nM), and all values have been obtained from multiple wells and expressed as mean ± SEM, and FIG. 3D illustrates the results of FACS analysis showing that the AbS0 antibody does not bind to the human ovarian cancer cell line SKOV3, which expresses L1CAM but does not express preS1 to exclude the possibility of non-specific binding of an AbS0 antibody to preS1;

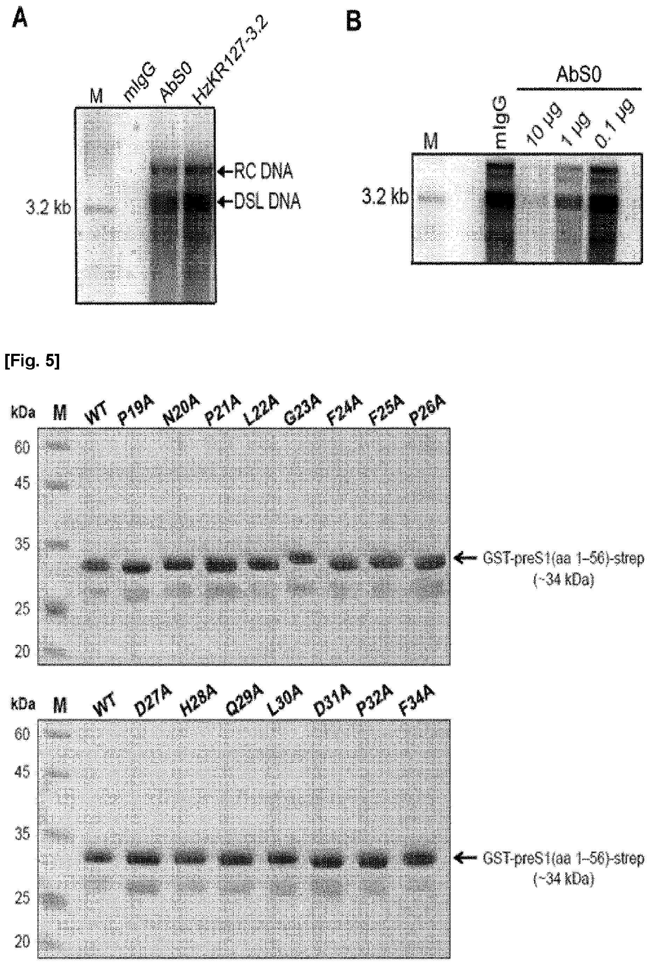

FIG. 4 illustrates the results of evaluating the virion binding activity (Fig. 4A) and *in-vitro* HBV-neutralizing activity (Fig. 4B) of AbS0 to HBV genotype D, FIG. 4A illustrates immunoprecipitation of HBV genotype D particles by AbS0, in which HzKR127-3.2 (ref: Kim JH et al. Enhanced humanization and affinity maturation of neutralizing anti-hepatitis B virus preS1 antibody based on antigen-antibody complex structure. FEBS Lett. 589:193-200) has been used as a positive control and mouse IgG as a negative control, RC DNA denotes relaxed circular DNA, and DSL DNA denotes double-stranded linear DNA, and FIG. 4B illustrates the results of extracting HBV DNA from harvested cells and performing Southern blot hybridization, in which HBV genotype D particles had been pre-incubated with AbS0 (10 µg, 1 µg, or 0.1 µg) or mouse IgG (10 µg) and then added to cultured HepG2-NTCP cells, the medium had been changed every 2 days, and the cells had been harvested 7 days after infection;
FIG. 5 illustrates the results of performing expression, purification, and SDS-PAGE of an alanine substitution mutant of amino acids 19 to 34 of GST-preS1(aa 1-56)-strep to map the epitope of an AbS0 antibody;

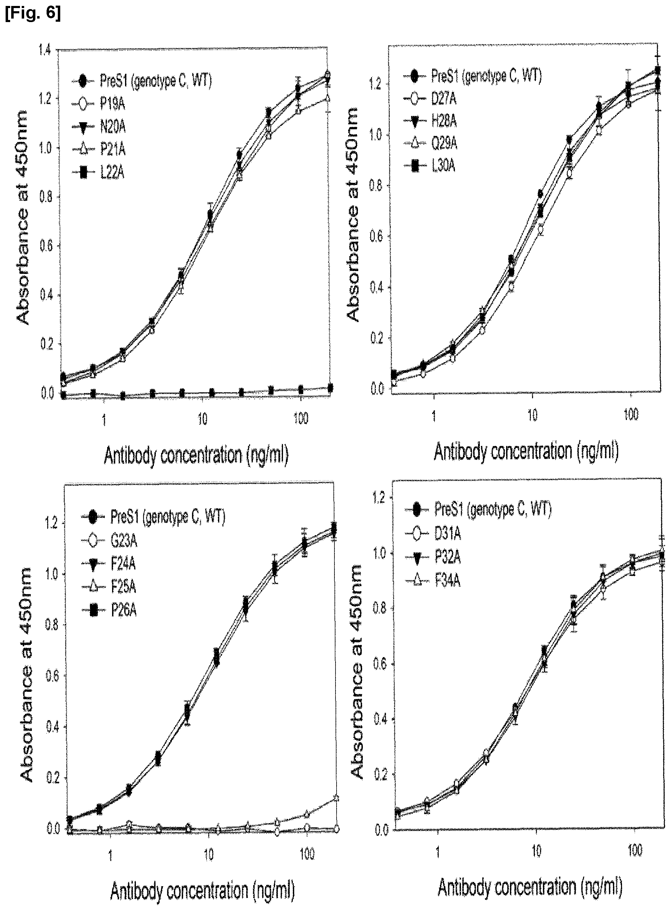

FIG. 6 illustrates the results of indirect ELISA to analyze the binding activity of an AbS0 antibody to an alanine substitution mutant of amino acids 19 to 34 of GST-preS1(aa 1-56)-strep;

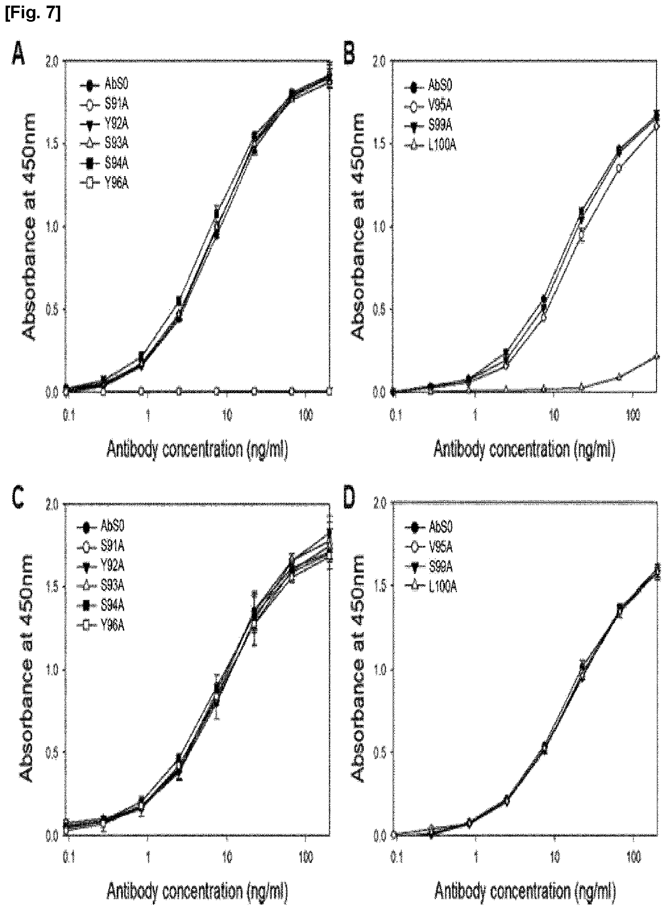

FIG. 7 illustrates the results of analyzing the antigen binding activities of antibody mutants in which each residue of LCDR3 (FIGS. 7A and 7C) and HCDR3 (FIGS. 7B and 7D) of an AbS0 antibody is substituted to alanine by indirect ELISA (7A and 7B) and quantitative ELISA (7C and 7D) to determine the binding site (paratope) of the AbS0 antibody for the preS1 antigen;

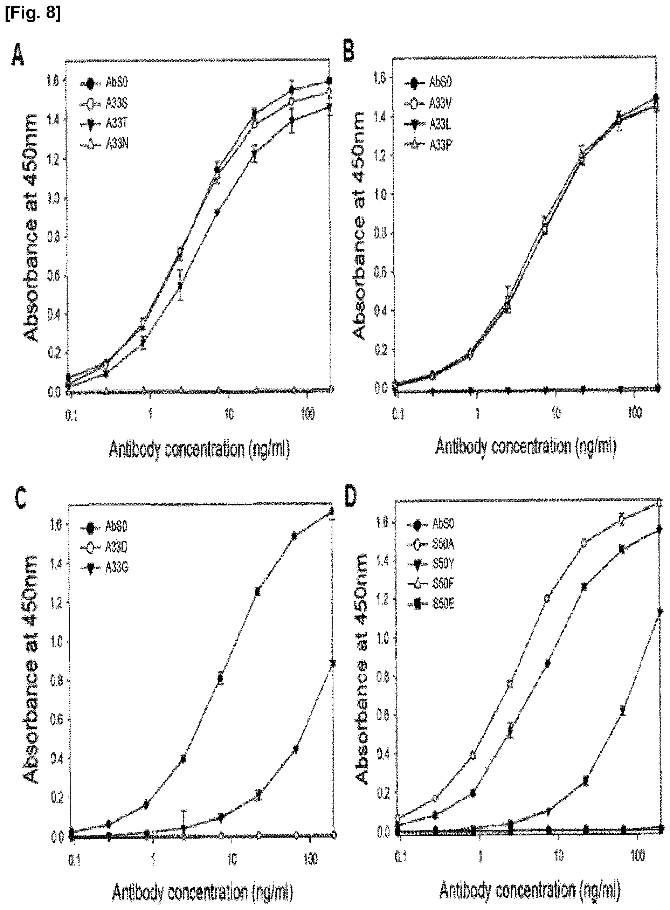

FIG. 8 illustrates the results of analyzing the antigen binding activities of antibody mutants in which Ala33 of HCDR1 (FIGS. 8A to 8C) and Ser50 of HCDR2 (FIG. 8D) of an AbS0 antibody are substituted to other residues by indirect ELISA to determine the binding site (paratope) of the AbS0 antibody for the preS1 antigen;

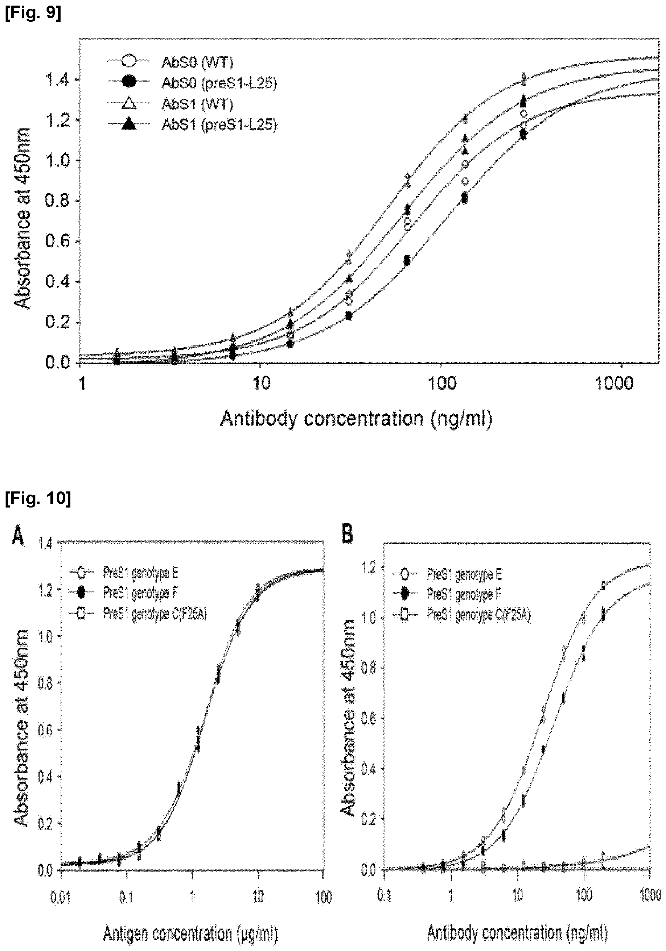

FIG. 9 illustrates the results of analyzing the antigen binding activities of AbS1 and AbS0 antibodies to GST-preS1(aa 1-56)-strep protein of genotype A having a conserved (NPLGFFP; WT) receptor binding motif or a receptor binding motif of another sequence (NPLGFLP; preS1-L25) by indirect ELISA; and
FIG. 10 illustrates the results of analyzing the antigen binding activities of an AbS1 antibody to GST-preS1(aa 1-56)-strep protein of genotypes E and F by quantitative ELISA (FIG. 10A) and indirect ELISA (FIG. 10B).

### [Detailed Description of the Invention]

Hereinafter, this application will be described in more detail. Meanwhile, each description and each embodiment disclosed herein may also be applied to other descriptions and embodiments. That is, all combinations of the various elements disclosed in this application fall within the scope of this application. Further, the scope of the present application is not limited by the specific description below. Additionally, a number of papers and patent documents are referenced and citations are indicated throughout this specification. The disclosures of the cited papers and patent documents are incorporated herein by reference in their entirety to more clearly explain the content of the present invention and the level of the technical field to which the present invention pertains.

In addition, those skilled in the art will recognize, or be able to ascertain using no more than routine experimentation, many equivalents to the specific aspects of the present invention described in this application. Additionally, such equivalents are intended to be encompassed by the present invention.

Throughout this specification, the customary one- and three-letter codes for naturally occurring amino acids are used. Additionally, amino acids abbreviated herein are described in accordance with the IUPAC-IUB nomenclature.

| | | | |
|---|---|---|---|
| Alanine | Ala, A | Arginine | Arg, R |
| Asparagine | Asn, N | Aspartic acid | Asp, D |
| Cysteine | Cys, C | Glutamic acid | Glu, E |
| Glutamine | Gln, Q | Glycine | Gly, G |
| Histidine | His, H | Isoleucine | Ile, I |
| Leucine | Leu, L | Lysine | Lys, K |
| Methionine | Met, M | Phenylalanine | Phe, F |
| Proline | Pro, P | Serine | Ser, S |
| Threonine | Thr, T | Tryptophan | Trp, W |
| Tyrosine | Tyr, Y | Valine | Val, V |

An aspect of the present invention for achieving the objects of the present invention provides a novel antibody that specifically binds to a hepatitis B virus preS1 antigen.

In the present invention, the term "antibody" refers to a protein molecule acting as a receptor that specifically recognizes an antigen, including immunoglobulin molecules that are immunologically reactive with a specific antigen, and includes polyclonal antibodies, monoclonal antibodies, whole antibodies, and antibody fragments.

The term also includes chimeric antibodies, humanized antibodies, human antibodies and bivalent or bispecific molecules (e.g., bispecific antibodies), diabodies, triabodies and tetrabodies. The term further includes single chain antibodies possessing a binding function to FcRn, scaps, derivatives of antibody constant regions, and artificial antibodies based on protein scaffolds. A whole antibody has a structure of two full-length light chains and two full-length heavy chains, and each light chain is connected to the heavy chain by a disulfide bond. The whole antibody includes IgA, IgD, IgE, IgM, and IgG, and IgG includes IgG1, IgG2, IgG3, and IgG4 as subtypes. The antibody fragment refers to a fragment that possesses an antigen binding function and includes Fd, Fab, Fab', F(ab')₂, and Fv. The Fd refers to the heavy chain portion contained in the Fab fragment. The Fab has a structure that includes variable regions of light and heavy chains, a constant region of the light chain, and the first constant region (CH1 domain) of the heavy chain, and has one antigen binding site. Fab' differs from Fab in that it has a hinge region containing one or more cysteine residues at the C-terminus of the heavy chain CH1 domain. The F(ab')₂ antibody is produced as cysteine residues in the hinge region of Fab' form a disulfide bond. Fv (variable fragment) refers to the minimum antibody fragment containing only a heavy chain variable region and a light chain variable region. In double disulfide Fv (dsFv), a heavy chain variable site and a light chain variable site are connected by a disulfide bond. In single chain Fv (scFv), a variable region of the heavy chain and a variable region of the light chain are generally connected by a covalent bond through a peptide linker. These antibody fragments can be obtained using proteolytic enzymes (for example, Fab can be obtained by restriction digestion of the whole antibody with papain, and F(ab')₂ fragment can be obtained by digestion with pepsin), and can specifically be constructed through genetic recombination technology.

The antibody of the present invention may be a monoclonal antibody.

In the present invention, the term "monoclonal antibody" refers to an antibody molecule of single molecular composition obtained from a substantially identical antibody population, and such a monoclonal antibody exhibits single binding specificity and affinity for a specific epitope.

Typically, immunoglobulins have heavy and light chains, and each heavy chain and each light chain contain a constant region and a variable region (the sites are also known as domains). The variable regions of the light and heavy chains include three hypervariable regions called complementarity-determining regions (hereinafter referred to as "CDRs") and four framework regions. The CDRs mainly function to bind to the epitope of an antigen. The CDRs of each chain are typically called CDR1, CDR2, and CDR3 sequentially starting from the N-terminus, and are also identified by the chain on which a particular CDR is located.

The antibody of the present invention may be a human antibody.

In the present invention, the term "human antibody" refers to a molecule derived from human immunoglobulin, and means that all amino acid sequences constituting the antibody, including the complementarity-determining regions and framework region, are composed of the amino acid sequence of human immunoglobulin. Human antibodies are commonly used in the treatment of human diseases, and may have at least three potential advantages. First, a human antibody interacts better with the human immune system, and can destroy target cells more efficiently, for example, by complement-dependent cytotoxicity (CDC) or antibody-dependent cell-mediated cytotoxicity (ADCC). Second, a human antibody has the advantage that the human immune system does not recognize the antibody as foreign. Third, a human antibody has the advantage that its half-life in the human circulatory system is similar to that of a naturally occurring antibody even when the drug is administered in smaller amounts and less frequently.

When the antibody of the present invention as described above contains a constant region, the antibody may contain a constant region derived from IgG, IgA, IgD, IgE, or IgM or by the combination or hybrid thereof.

In the present invention, the term "combination" means that a polypeptide encoding a single-chain immunoglobulin constant region of the same origin forms a bond with a single-chain polypeptide of a different origin when forming a dimer or multimer. For example, a dimer or multimer may be formed from two or more constant regions selected from the group consisting of constant regions of IgG, IgA, IgD, IgE, and IgM.

In the present invention, the term "hybrid" means that sequences corresponding to two or more immunoglobulin heavy chain constant regions of different origins exist within a single-chain immunoglobulin heavy chain constant region, and for example, a domain hybrid consisting of 1 to 4 domains selected from the group consisting of CH1, CH2, CH3, and CH4 of IgG, IgA, IgD, IgE, and IgM is possible.

In the present invention, the term "hepatitis B virus" is used interchangeably with HBV, and refers to a virus that can cause hepatitis B due to an immune response when infected.

In the present invention, the term "preS1 antigen" refers to a protein encoded by the S gene, one of the four genes S, C, X, and P of HBV DNA. Specifically, the S gene is a gene that encodes a surface antigen, and has a Pre-S region in front of it, and Pre-S is divided into a PreS1 region and a PreS2 region. These encode the surface proteins, S (small) protein, M (middle) protein, and L (large) protein. The S protein is encoded by the S gene, the M protein is composed of preS2 and the S protein, and the L protein is composed of preS1, preS2, and the S protein and is mainly present in infectious virus particles (virions). Therefore, preS1 constitutes the surface protein of a virus and is mainly present in infectious virions.

The antibody provided by the present invention may bind to diverse genotypes of HBV, and specifically may bind to all genotypes A, B, C, D, E, and F. In an embodiment, the antibody provided by the present invention can bind to the receptor binding motif sequence shared by genotypes A, B, C, D, E, and F of preS1, but is not limited thereto.

In the present invention, the term "antibody that binds to the hepatitis B virus preS1 antigen" refers to an antibody that binds to preS1, the surface antigen of HBV, and can exhibit neutralizing activity and/or HBV infection-inhibiting activity.

In an embodiment, the antibody provided by the present invention may bind to an amino acid at a specific position of the preS1 antigen, and the position may be positions corresponding to positions 22, 23, and 25 based on SEQ ID NO: 19.

The SEQ ID NO: 19 is only a reference sequence for specifying the binding position of the antibody of the present invention in any preS1 antigen, and the preS1 antigen of the present invention is not limited to SEQ ID NO: 19. In any preS1 antigen protein, positions corresponding to positions 22, 23, and 25 based on SEQ ID NO: 19 can be confirmed through sequence alignment. For such sequence alignment, the Needleman-Wunsch algorithm, the Needle program of the EMBOSS package, multiple sequence alignment, and the like known in the art may be used, but the method is not limited thereto.

The amino acid sequence of the preS1 antigen can be obtained from a known database, and may include, for example, the sequence of SEQ ID NO: 17 or 18. Amino acids 22, 23, and 25 may be amino acids represented by numbers 3, 4, and 6, respectively, in the amino acid sequence of SEQ ID NO: 17 or 18, but are not limited thereto.

Amino acids 22, 23, and 25 based on SEQ ID NO: 19 are the sites where the antibody of the present invention binds, and the "epitope" of the preS1 antigen recognized by the antibody of the present invention can be expressed as positions corresponding to positions 22, 23, and 25 based on SEQ ID NO: 19. Meanwhile, positions corresponding to positions 22, 23, and 25 based on SEQ ID NO: 19 may be included in the site where the preS1 antigen binds to the hepatocyte receptor. Therefore, the antibody of the present invention can inhibit binding of the preS1 antigen to the hepatocyte receptor.

In an embodiment according to any one of the above-described embodiments, the antibody may bind to leucine (Leu22) corresponding to position 22, glycine (Gly23) corresponding to position 23, and phenylalanine (Phe25) or leucine (Leu25) corresponding to position 25 based on SEQ ID NO: 19.

In an embodiment according to any one of the above-described embodiments, the antibody may be an antibody having tyrosine 8 of the LCDR3 sequence of SEQ ID NO: 5 and leucine 6 of the HCDR3 sequence of SEQ ID NO: 9 as paratopes.

In an embodiment according to any one of the above-described embodiments, the antibody may be an antibody having amino acid 1 of the HCDR2 sequence represented by SEQ ID NO: 8 as a paratope. Amino acid 1 of SEQ ID NO: 8 may be alanine or leucine.

In the present invention, the term "paratope" refers to a region or zone on an antibody or antibody fragment in which the antibody or antibody fragment specifically binds to an antigen, that is, the antibody or antibody fragment physically contacts with the antigen.

In an embodiment according to any one of the above-described embodiments, the antibody may be an antibody that specifically binds to preS1, containing a light chain variable region including light chain CDR1 (LCDR1) of SEQ ID NO: 3, light chain CDR2 (LCDR2) of SEQ ID NO: 4, and light chain CDR3 (LCDR3) of SEQ ID NO: 5; and a heavy chain variable region including heavy chain CDR1 (HCDR1) of SEQ ID NO: 7, heavy chain CDR2 (HCDR2) of SEQ ID NO: 8, and heavy chain CDR3 (HCDR3) of SEQ ID NO: 9.

In an embodiment according to any one of the above-described embodiments, the antibody may be an antibody that specifically binds to preS1, containing a light chain variable region including light chain CDR1 (LCDR1) of SEQ ID NO: 3, light chain CDR2 (LCDR2) of SEQ ID NO: 4, and light chain CDR3 (LCDR3) of SEQ ID NO: 6; and a heavy chain variable region including heavy chain CDR1 (HCDR1) of SEQ ID NO: 10, heavy chain CDR2 (HCDR2) of SEQ ID NO: 11 or 12, and heavy chain CDR3 (HCDR3) of SEQ ID NO: 13.

In an embodiment according to any one of the above-described embodiments, the light chain variable region of the antibody may be composed of the amino acid sequence represented by SEQ ID NO: 1. The light chain variable region of the antibody may be specifically composed of the amino acid sequence represented by SEQ ID NO: 14, but is not limited thereto.

In an embodiment according to any one of the above-described embodiments, the heavy chain variable region of the antibody may be composed of the amino acid sequence represented by SEQ ID NO: 2. The heavy chain variable region of the antibody may be specifically composed of the amino acid sequence represented by SEQ ID NO: 15 or 16, but is not limited thereto.

In an embodiment according to any one of the above-described embodiments, the antibody may contain a light chain variable region represented by SEQ ID NO: 1 and a heavy chain variable region represented by SEQ ID NO: 2, but is not limited thereto. The antibody may specifically contain a light chain variable region represented by SEQ ID NO: 14; and a heavy chain variable region represented by SEQ ID NO: 15 or 16, but is not limited thereto.

In an embodiment of the present invention, the antibody that specifically binds to the preS1 region, containing the light chain variable region of SEQ ID NO: 14 and the heavy chain variable region of SEQ ID NO: 15, has been named AbS0, and it has been confirmed that AbS0 exhibits excellent binding ability to all HBV genotypes A, B, C, and D.

It has been confirmed that tyrosine 8 of the LCDR3 sequence of SEQ ID NO: 6, leucine 6 of the HCDR3 sequence of SEQ ID NO: 13, and serine 1 of the HCDR2 sequence of SEQ ID NO: 11 are paratopes of the AbS0 antibody. As a result of substituting serine 1 of HCDR2 in the AbS0 antibody with another amino acid (alanine), it has been confirmed that an antibody having the HCDR2 sequence represented by SEQ ID NO: 12 is superior to AbS0 in binding ability, and the antibody having the HCDR2 sequence of SEQ ID NO: 12 has been named AbS1 antibody.

Such a human antibody binding to preS1 of the present invention affords higher affinity and lower immunogenicity compared to existing antibodies and can exhibit excellent neutralizing and infection-inhibiting activity against HBV. The antibody of the present invention can thus be used in any application where antigen recognition against preS1, neutralization of HBV, and inhibition of HBV infection can be usefully used.

Another aspect of the present invention provides a method for producing the antibody.

The antibody of the present invention can be easily produced using known antibody production techniques. For example, the method for producing monoclonal antibodies can be performed by producing hybridomas using B lymphocytes obtained from immunized animals (Koeher and Milstein, 1976, Nature, 256:495), by using phage display technology, or the like, but is not limited thereto. The antibody of the present invention can be easily produced using other known antibody production techniques.

The antibody library using phage display technology is a method of directly obtaining antibody genes from B lymphocytes without constructing hybridomas and expressing antibodies on the surface of phage. By the use of phage display technology, a number of the existing difficulties associated with the production of monoclonal antibodies by B-cell immortalization can be overcome. In general, phage display technology may include the steps of 1) inserting an oligonucleotide of a random sequence into a gene site corresponding to the N-terminus of the coat protein pill of a phage; 2) expressing a fusion protein of a portion of the native coat protein and a polypeptide encoded by the oligonucleotide of a random sequence; 3) treating a material capable of binding to the polypeptide encoded by the oligonucleotide; 4) eluting the antibody-phage particles bound to the material using low pH or a binding competitive molecule; 5) amplifying the phages eluted by panning within a host cell; 6) repeating the method to obtain the desired amount; and 7) determining the sequence of an active antibody from the DNA sequences of phage clones selected by panning.

The method for producing a monoclonal antibody of the present invention can be performed using phage display technology. Those skilled in the art can easily perform each step of the production method of the present invention by referring to known phage display technologies, for example, methods known in papers, such as Barbas et al. (METHODS: A Companion to Methods in Enzymology 2:119, 1991 and J. Virol. 2001 Jul;75(14):6692-9) and Winter et al. (Ann. Rev. Immunol. 12:433, 1994). Phages that can be used to construct an antibody library include, for example, filamentous phages such as fd, M13, f1, If1, Ike, Zj/Z, Ff, Xf, Pf1, or Pf3 phage, but are not limited thereto. Vectors that can be used for expression of heterologous genes on the surface of filamentous phages include, for example, phage vectors such as fUSE5, fAFF1, fd-CAT1 or fdtetDOG, or phagemid vectors such as pHEN1, pComb3, pComb8 or pSEX, but are not limited thereto. Helper phages that can be used to afford the wild-type coat protein required for successful reinfection of recombinant phage for amplification include, for example, M13K07 or VSCM13, but are not limited thereto.

Polynucleotides encoding monoclonal antibodies or phage display clones of the present invention can be easily isolated and sequenced using routine procedures. As an example, oligonucleotide primers designed to specifically amplify the heavy chain and light chain coding regions from phage template DNA can be used. Once the polynucleotide is isolated, this can be placed in an expression vector, then the expression vector can be introduced into an appropriate host cell, and the desired monoclonal antibody can be produced from the transformed host cell (*i.e.*, transformant). Therefore, the method for producing a human monoclonal antibody of the present invention may be a method for producing a human monoclonal antibody, which includes amplifying a polynucleotide encoding a human monoclonal antibody in an expression vector containing the polynucleotide encoding the human monoclonal antibody, but is not limited thereto.

Still another aspect of the present invention provides a polynucleotide encoding the antibody, an expression vector containing the polynucleotide, and a host cell into which the vector is introduced.

The antibody is as described above.

The expression vector containing a polynucleotide encoding the antibody provided by the present invention may be a vector capable of replicating and/or expressing the polynucleotide in eukaryotic or prokaryotic cells, including mammalian cells (*e.g.*, human, monkey, rabbit, rat, hamster, and mouse cells), plant cells, yeast cells, insect cells, or bacterial cells (e.g., *E. coli*), but is not particularly limited thereto. Specifically, the expression vector may be a vector that is operably linked to an appropriate promoter so that the nucleotide can be expressed in a host cell and contains at least one selection marker. The expression vector may be, for example, in a form in which the polynucleotide is introduced into a phage, plasmid, cosmid, mini-chromosome, virus, or retroviral vector.

The expression vector containing a polynucleotide encoding the antibody may be an expression vector containing a polynucleotide encoding the heavy chain of the antibody or a polynucleotide encoding the light chain of the antibody or an expression vector containing both a polynucleotide encoding the heavy chain of the antibody and a polynucleotide encoding the light chain of the antibody.

The host cell into which the expression vector provided by the present invention is introduced may be cells of bacteria such as *Escherichia coli*, *Streptomyces*, or *Salmonella typhimurium*; yeast cells; fungal cells such as *Pichia pastoris*; insect cells such as *Drozophila* and *Spodoptera Sf9* cells; animal cells such as CHO (Chinese hamster ovary cells), SP2/0 (mouse myeloma), human lymphoblastoid, COS, NSO (mouse myeloma), Bowes melanoma cells, HT-1080, BHK (baby hamster kidney cells), HEK (human embryonic kidney cells), and PERC.6 (human retina cells); or plant cells, which are transformed by introduction of the expression vector, but is not particularly limited thereto.

In the present invention, the term "introduction" refers to a method of delivering a vector containing a polynucleotide encoding the antibody to a host cell. Such introduction can be performed by various methods known in the art, such as calcium phosphate-DNA co-precipitation method, DEAE-dextran-mediated transfection method, polybrene-mediated transfection method, electric shock method, microinjection method, liposome fusion method, and lipofectamine and protoplast fusion method. Transduction means delivering a target into a cell using virus particles through infection. In addition, vectors can be introduced into host cells by gene gun bombardment, and the like. In the present invention, introduction can be used interchangeably with transformation.

Still another aspect of the present invention provides a pharmaceutical composition for prevention or treatment of HBV infection, containing the antibody. Specifically, the composition may be for prevention or treatment of hepatitis B.

In the present invention, the term "hepatitis B" refers to a disease in which liver inflammation occurs due to an immune response when the liver is infected with HBV. The antibody of the present invention exhibits excellent neutralizing and infection-inhibiting ability against HBV, and can be thus used for the prevention or treatment of hepatitis B.

In the present invention, the term "prevention" may mean any action that suppresses or delays the onset of HBV infection by administering the composition, and the term "treatment" may mean any action that improves or beneficially changes symptoms caused by HBV infection by administering the composition.

The pharmaceutical composition may further contain a pharmaceutically acceptable carrier.

In the present invention, the term "pharmaceutically acceptable carrier" refers to a carrier or diluent that does not irritate living organisms and does not inhibit the biological activity and properties of the administered compound. As acceptable pharmaceutical carriers in compositions formulated as liquid solutions, saline solution, sterile water, Ringer's solution, buffered saline solution, albumin injection solution, dextrose solution, maltodextrin solution, glycerol, and ethanol, which are sterile and biocompatible, and one or more of these components can be used in mixture. If necessary, other common additives such as antioxidants, buffers, and bacteriostatic agents can be added. In addition, diluents, dispersants, surfactants, binders, and lubricants can be additionally added to prepare injectable formulations such as aqueous solutions, suspensions, and emulsions, pills, capsules, granules, or tablets.

The pharmaceutical composition may be in various oral or parenteral formulations. When the pharmaceutical composition is formulated into a preparation, the preparation is prepared using diluents or excipients such as commonly used fillers, extenders, binders, wetting agents, disintegrants, and surfactants. Solid preparations for oral administration include tablets, pills, powders, granules, capsules, and the like, and these solid preparations are prepared by mixing one or more compounds with at least one or more excipients, such as starch, calcium carbonate, sucrose or lactose, and gelatin. In addition to simple excipients, lubricants such as magnesium stearate and talc are also used. Liquid preparations for oral administration include suspensions, oral solutions, emulsions, and syrups. In addition to the commonly used simple diluents such as water and liquid paraffin, various excipients, such as wetting agents, sweeteners, fragrances, and preservatives may be contained. Preparations for parenteral administration include sterilized aqueous solutions, non-aqueous solutions, suspensions, emulsions, freeze-dried preparations, and suppositories. As non-aqueous solvents and suspensions, propylene glycol, polyethylene glycol, vegetable oil such as olive oil, and injectable esters such as ethyl oleate may be used. As a base for suppositories, Witepsol, Macrogol, Tween 61, cacao oil, laurin oil, glycerogelatin, and the like may be used.

The pharmaceutical composition may be in any one formulation selected from the group consisting of tablets, pills, powders, granules, capsules, suspensions, oral solutions, emulsions, syrups, sterilized aqueous solutions, non-aqueous solvents, suspensions, emulsions, freeze-dried preparations, and suppositories.

The composition of the present invention is administered in a pharmaceutically effective amount.

In the present invention, the term "pharmaceutically effective amount" means an amount sufficient to treat a disease with a reasonable benefit/risk ratio applicable to medical treatment, and the effective dose level may be determined based on factors including the type and severity of the subject, age, sex, type of cancer, activity of the drug, sensitivity to the drug, time of administration, route of administration and excretion rate, duration of treatment, and drugs used concurrently, and other factors well known in the medical field. The composition of the present invention may be administered as an individual therapeutic agent or in combination with other therapeutic agents, and may be administered sequentially or simultaneously with conventional therapeutic agents. The composition of the present invention may be administered singly or multiple times. Considering all of the factors, it is important to administer the composition in an amount in which the maximum effect can be achieved with the minimum amount without side effects, and the amount can be easily determined by those skilled in the art.

Still another aspect of the present invention provides a method for preventing or treating HBV infection using the antibody.

Specifically, the method may include administering the antibody to a subject suspected of being infected with HBV.

The method may be a method for preventing or treating HBV infection, which includes administering a pharmaceutical composition containing an antibody and further a pharmaceutically acceptable carrier to a subject to be infected or infected with HBV. The pharmaceutically acceptable carrier is as described above.

The subject includes mammals, including cows, pigs, sheep, chickens, dogs, humans, and the like, birds, and the like, and includes subjects whose HBV infection is treated by administration of the composition of the present invention without limitation. At this time, the antibody may be administered singly or multiple times in a pharmaceutically effective amount. At this time, the antibody can be administered in the form of a liquid, powder, aerosol, capsule, enteric-coated tablet or capsule, or suppository. The route of administration includes intraperitoneal administration, intravenous administration, intramuscular administration, subcutaneous administration, endothelial administration, oral administration, topical administration, intranasal administration, intrapulmonary administration, intrarectal administration, and the like, but is not limited thereto.

The method can also be used to prevent or treat hepatitis B, and may be specifically used to treat HBV infection with any one or more selected from HBV genotypes A, B, C, D, E, and F, but is not limited thereto.

Still another aspect of the present invention is a use of the antibody to be used in the manufacture of a medicine for prevention or treatment of HBV infection. The medicine can also be used to prevent or treat hepatitis B, and can be used to treat HBV infection with any one or more selected from HBV genotypes A, B, C, D, E, and F, but is not limited thereto. The antibody and prevention or treatment of HBV infection are as previously described.

Still another aspect of the present invention provides a method of providing information for diagnosis of HBV infection, which includes detecting preS1 present in a biological sample isolated from a subject suspected of being infected with HBV through an antigen-antibody reaction using the antibody.

The method may be a method for diagnosing HBV infection. Additionally, the method can be used for diagnosis of hepatitis B.

The antibody and HBV are as previously described.

The method of providing information for diagnosis of HBV can detect a preS1 protein by reacting the antibody specific for preS1 of the present invention with a biological sample isolated from a subject suspected of being infected with HBV and detecting formation of an antigen-antibody complex, and through this, information for diagnosis of HBV infection can be provided.

Specifically, the method may be a method including (a) treating a biological sample isolated from a subject suspected of being infected with HBV with the antibody and detecting a preS1 through an antigen-antibody reaction; and (b) comparing the level of preS1 detected in (a) with that of the control group and determining the subject as a patient infected with HBV when the level of preS1 is higher than that of the control group.

In the present invention, the term "biological sample" includes tissues, cells, whole blood, serum, plasma, tissue autopsy samples (brain, skin, lymph nodes, spinal cord, and the like), cell culture supernatants, ruptured eukaryotic cells, and bacterial expression systems, but is not limited thereto. These biological samples can be reacted with the antibody of the present invention with or without manipulation to examine the presence of preS1 or HBV infection.

In the present invention, the term "antigen-antibody complex" refers to a combination of a preS1 antigen in a sample and the antibody according to the present invention that recognizes the antigen, and the formation of this antigen-antibody complex can be detected by any method by any method selected from the group consisting of colorimetric method, electrochemical method, fluorimetric method, luminometry, particle counting method, visual assessment, and scintillation counting method. However, the detection method is not necessarily limited to these, and various applications are possible.

In the present invention, various markers can be used to detect the antigen-antibody complex. As specific examples, the markers may be selected from the group consisting of enzymes, fluorescent substances, ligands, luminescent substances, microparticles, and radioactive isotopes, but are not necessarily limited thereto.

Enzymes used as detection markers include acetylcholinesterase, alkaline phosphatase, β-D-galactosidase, horseradish peroxidase, β-latamase, and the like, fluorescent substances include fluorescein, Eu³⁺, Eu³⁺ chelate, or cryptate, ligands include biotin derivatives, luminescent substances include acridinium ester and isoluminol derivatives, microparticles include colloidal gold and colored latex, and radioactive isotopes include ⁵⁷Co, ³H, ¹²⁵I, ¹²⁵I-Bonton Hunter reagent, and the like, but the detection markers are not limited thereto.

Specifically, the detection method may be one using an enzyme-linked immunosorbent assay (ELISA) for an antigen-antibody complex. The enzyme-linked immunosorbent assay (ELISA) includes various ELISA methods, such as direct ELISA using a labeled antibody that recognizes an antigen attached to a solid support, indirect ELISA using a labeled secondary antibody that recognizes a capture antibody in a complex of antibodies that recognize an antigen attached to a solid support, direct sandwich ELISA using another labeled antibody that recognizes an antigen in a complex of an antibody and an antigen attached to a solid support, and indirect sandwich ELISA using a labeled secondary antibody that recognizes an antibody after reacting the antibody with another antibody that recognizes an antigen in a complex of an antibody and the antigen attached to a solid support.

The antibody may have a detection label, and the antigen-antibody complex can be confirmed by treating another antibody that can capture these monoclonal antibodies and has a detection label when the antibody does not have a detection label.

Still another aspect of the present invention provides a composition for detection of HBV, containing the antibody provided by the present invention. The antibody is as described above.

The HBV detection may be detecting the preS1 of HBV.

In an embodiment, the composition may further contain tools, devices, or reagents that are contained in conventional compositions for detection to confirm the presence of an antigen from a biological sample using an antibody, such as tools for collecting samples to be tested or applying detection reagents and reagents or devices for confirming antigen-antibody binding.

Still another aspect of the present invention provides a kit for detection of HBV, containing the antibody. The HBV detection is as described above.

In an embodiment, the kit may also be in the form of a hepatitis diagnostic kit. The composition, hepatitis B, and diagnosis are as previously described.

Still another aspect of the present invention is a use of the antibody to be used in the production of a composition for detection of HBV. The antibody and HBV are as described above.

### [Examples]

Hereinafter, the present invention will be described in more detail through Examples and Experimental Examples. However, these Examples and Experimental Examples are for illustrative purposes only and the scope of the present invention is not limited to these Examples and Experimental Examples.

### Example 1: Construction of PreS1 antigen

Using the methods disclosed in Biotechnology letters. 17:871-876 (Kim HS, Hong HJ. 1995. Efficient expression, purification and characterization of hepatitis B virus preS1 protein from Escherichia coli) and the like, GST-preS1(1-119), GST-preS1(1-56), and preS1(1-119) of HBV genotype C were constructed. The biotinylated synthetic preS1 peptide (biotin-SGSGNPLGFFFPDHQLDP, Bio-preS1-L peptide) containing the NTCP binding motif (20-NPLGFFP-26) was produced by AnyGen, Inc. (South Korea) for use. The recombinant preS1 antigens and Bio-preS1-L peptide constructed in this way were used as antigens for panning of the antibody library. In order to analyze whether the discovered antibodies bind to the preS1 antigen of diverse HBV genotypes, GST-preS1(1-56)-strep tag of HBV genotypes A to F was expressed in E. *coli* and purified. The sequences of preS1(1-56) of genotypes A to F are illustrated in FIG. 1.

### Example 2: Selection of Fab specific to hepatocyte receptor binding motif of preS1 from phage displayed human synthetic Fab library

In order to isolate Fab specific to the hepatocyte receptor binding motif of HBV preS1, a human synthetic Fab phage display library was panned against four preS1 antigens.

Specifically, the human synthetic Fab library (diversity 1.35 × 10⁹) was panned against the four preS1 antigens GST-preS1(1-119), GST-preS1(1-56), Bio-preS1-L peptide and preS1(1-119) of Example 1 for 1st, 2nd, 3rd, and 4th rounds, respectively, according to a standard panning procedure. Each preS1 antigen was coated on an immunotube (streptavidin-coated plate for Bio-preS1-L peptide) and stored at 4°C overnight. Antibody library phages were incubated with the antigen, and unbound phages were washed off with 0.1% PBST (0.1% Tween 20 in PBS). Bound phages were eluted using 10 µg/mL trypsin solution at 37°C for 30 minutes. *E. coli* TG1 cells were infected with the eluted phages, grown at 37°C for 1 hour, and incubated at 37°C overnight. The next round of panning was performed with the amplified phage, and the degree of washing was gradually strengthened in each round. For each round, the input and output of phage numbers were calculated by dilution and CFU (FIG. 2A).

In order to examine whether antibody clones against the preS1 antigen are amplified as panning progressed, indirect ELISA was performed on the output phages of each round, and as a result, it was confirmed that Fab clones binding to the recombinant preS1 antigen and Bio-preS1-L peptide were amplified after the third round of panning (FIG. 2B).

Therefore, in order to select Fab clones exhibiting the highest activity to the preS1 antigen, 188 phage Fab clones were randomly selected after the fourth round of panning, and indirect ELISA was performed using the Bio-preS1-L peptide, which was common to the preS1 antigen of HBV genotypes (A to D).

Specifically, after fourth round of panning was performed, a total of 188 colonies were randomly selected, cultured, and then infected with helper phages to obtain monoclonal Fab phages. Afterwards, indirect ELISA was performed on these phages to confirm positive clones. In indirect ELISA, 100 ng of BSA or Bio-PreS1-L peptide were used as immobilization antigens, and horseradish peroxidase (HRP)-conjugated anti-M13 (1:5000 v/v, GE Healthcare) was used as a secondary antibody. In quantitative ELISA, anti-M13 antibody (100 ng/well, GE Healthcare) and anti-human kappa HRP (1:2000 v/v, Novex) were used as an immobilized antibody and a secondary antibody, respectively.

As a result of the screening, it was confirmed that the AbS0 Fab phage exhibited the highest antigen binding activity to the Bio-PreS1-L peptide, and also exhibited high binding activity to the preS1(1-119) antigen of Example 1 (FIG. 2C).

### Example 3: Conversion of AbS0 Fab into human IgG1 and analysis of antigen binding activity

AbS0 Fab was converted into human IgG1 and expressed in HEK293F cells, and the culture supernatant was used for protein purification.

### 3-1 Conversion of AbS0 Fab to human IgG1

In order to convert the selected Fab into an IgG1 form, the heavy chain variable region (VH) and kappa light chain variable region (VK) sequences were amplified by PCR and then combined with the IgG heavy and light chain leader sequences, respectively, using recombinant PCR. The resulting VH and VK sequences were subcloned into the *EcoRI-Apal* and *HindIII-BsiWI* sites of the pdCMV-dhfrC expression plasmid containing human Cy1 and C_{K} genes to construct a pdCMV-dhfrC-AbS0 expression plasmid.

### 3-2 Antibody purification

The expression plasmid constructed in 3-1 was introduced into 30 mL of HEK293F cells at a ratio of 1:4 (30 µg : 120 µg) using polyethyleneimine (PEI). The transformed cells were cultured for 7 days, and the culture supernatant was subjected to affinity chromatography using Protein A, then the concentration of the antibody was determined using a UV-Vis Spectrophotometer. The purity of the purified antibody was verified by SDS-PAGE (FIG. 3A).

### 3-3 Analysis of binding activity

In order to analyze the antigen binding activity of purified AbS0 mAb to the preS1 antigen of diverse HBV genotypes, each well was coated with GST-preS1(1-56)-strep (30 nM/well) of genotypes A to D (FIG. 1) and then incubated with the serially diluted AbS0 antibody. The bound AbS0 antibody was detected using anti-human IgG Fc-HRP (1:10,000 v/v, Jackson) as a secondary antibody.

As a result, it was confirmed that the purified AbS0 antibody bound well to all recombinant preS1 of genotypes A to D (FIG. 3B). Through this, it has been confirmed that the AbS0 antibody exhibits binding activity to the preS1 antigen of genotypes A to D.

### 3-4 Analysis of antigen binding affinity of AbS0

The affinity of the antibody was determined by Bio-Layer Interferometry using Octet RED384 (ForteBio). Specifically, anti-human Fc-coated biosensor tips (AHC, ForteBio) were activated by stirring a 96-well microtiter plate (Greiner Bio-One) in 0.1% PBA (0.1% BSA in PBS) at 1000 rpm for 20 minutes. The AbS0 antibody (1 µg/mL, 200 µL) was added and captured for 10 minutes, and washing with 0.1% PBA was performed for 2 minutes. The GST-preS1(1-56)-strep antigen of HBV genotype A was subjected to 2-fold serial dilution (25 nM, 12.5 nM, 6.25 nM, 3.125 nM, and 1.5625 nM) with 0.1% PBA, and subjected to an association step and a dissociation step with the antibody bound to the tip for 5 minutes and 10 minutes, respectively. For baseline drift, the measured values were corrected by subtracting the value of the control sensor (antibody-captured AHC sensor) exposed only to the running buffer without containing the antibody. The operating temperature was maintained at 30°C. Data were analyzed using ForteBio data analysis software version 7.1 using a 1:1 interaction model (fitting global, Rmax unlinked by sensor).

As a result, it was confirmed that the affinity of the AbS0 antibody was 0.5 nM.

**[Table 1]**

| Antibody affinity measured by Bio-Layer Interferometry using Octet RED384 (ForteBio) | | | | |
|---|---|---|---|---|
| **Antibody** | **K_{D}** | **Kₒₙ (1/Ms)** | **K_{dis} (1/s)** | **Full R²** |
| AbS0 | 0.50 nM | 3.92 × 10⁵ | 1.94 × 10⁻⁴ | 0.9969 |

| | | | | |
|---|---|---|---|---|
| *Kₒₙ, rate of association; K_{dis}, rate of dissociation; Full R², estimate of the goodness of the curve fit | | | | |

### 3-5 Evaluation of antigen binding specificity of AbS0 antibody

In order to exclude the possibility of non-specific binding of the AbS0 antibody to a preS1 antigen, flow cytometry was performed using the human ovarian cancer cell line SKOV3 (Min, J.-K. et al., Clin. Cancer Res. 16(14);3571-80, 2010), which expressed L1 CAM but did not express preS1.

Specifically, 1 µg (in 100 µL of 0.1 % PBA) of AbS0 or Ab417 (L1CAM-specific human antibody: Cho S. et al., MABS 8(2), 414-425, 2016) as a positive control was added to cultured SKOV3 cells (2 × 10⁵), the reaction was conducted at 4°C for 1 hour, and then washing with 0.1% PBA was performed twice. Anti-human IgG Fc-fluorescein isothiocyanate (FITC) conjugate (1:2000 v/v, BD Pharmingen) was added to these cells, the reaction was conducted at 4°C for 1 hour, and then propidium iodide (1:200 v/v, Sigma) was added. Finally, the cells were analyzed using FACSCalibur (Becton Dickinson). As a result of the experiment, Ab417 bound to SKOV3, but the AbS0 antibody did not bind to the SKOV3 cells (FIG. 3D).

Through this, it has been confirmed that the AbS0 antibody specifically binds to the preS1 antigen.

### Example 4: Evaluation of HBV-neutralizing activity of AbS0 antibody

### 4-1 Immunoprecipitation analysis

In order to examine whether the AbS0 antibody binds to HBV virions, immunoprecipitation analysis was performed using HBV genotype D.

The existing anti-preS1 humanized antibody HzKR127-3.2, which exhibited HBV-neutralizing efficacy, was used as a positive control for HBV genotype D, and mouse IgG was used as a negative control. After immunoprecipitation, viral DNA was extracted and measured by Southern blot hybridization.

Specifically, in order to produce HBV particles of HBV genotype D, HepG2 cells were seeded in a 6-well plate at a density of 6 × 10⁵ and cultured at 37°C. The next day, the HepG2 cells were transfected with pHBV1.2 (HBV genotype D) and incubated for 4 days. The culture supernatant was harvested and incubated with 1 µg/mL of AbS0, HzKR127-3.2 (positive control), or mouse IgG (negative control) at 4°C overnight, and then immunoprecipitation was performed with 20 µL of protein A beads at 4°C for 6 hours. The immunoprecipitated complex was washed three times with PBS, and then viral DNA was detected as follows. Specifically, in order to remove the transfected plasmid DNA, the immune complex was treated with DNase I (Sigma) and mung bean nuclease (Takara, Kusatsu, Shiga, Japan) at 37°C for 20 minutes. Next, in order to obtain core-associated HBV DNA, the precipitated immune complex was digested by adding Proteinase K (20 mg/mL, Roche) at 37°C for 2 to 3 hours in the presence of 0.5% SDS, then the HBV DNA was extracted using phenol/chloroform/isoamyl alcohol (25:24:1) (Sigma) and purified using ethanol and 3 M sodium acetate. The purified DNA was separated on a 1% agarose gel, and HBV DNA was detected by Southern blot hybridization.

As a result, RC (relaxed circular) and DSL (double-stranded linear) forms of HBV DNA were detected in AbS0 or HzKR127-3.2 antibody precipitates, whereas HBV DNA was not detected in immunoprecipitation with mouse IgG (FIG. 4A).

Through this, it has been confirmed that the AbS0 antibody can bind to virions of HBV genotype D.

### 4-2 Evaluation of HBV-neutralizing activity of AbS0

In order to test whether AbS0 can neutralize HBV infection, HBV particles of genotype D were pre-incubated with antibodies at different concentrations, and added to HepG2-NTCP cells overexpressing cell receptor NTCP.

Specifically, the HBV-neutralizing activity of AbS0 was analyzed using the HepG2 (referred to as HepG2-NTCP) cell line overexpressing NTCP. HepG2-NTCP cells were seeded in a 6-well plate at a density of 6 × 10⁵ and cultured at 37°C. The next day, the HepG2-NTCP cells were infected (approx. 2000 viral genome equivalents per cell) with HBV particles of genotype D in primary hepatocyte maintenance medium (PMM) containing 4% polyethylene glycol (PEG) and 2.5% DMSO.

For neutralization assay, HBV particles were pre-incubated with diluted AbS0 antibody (10 µg, 1 µg, or 0.1 µg) or mouse IgG (10 µg) for 1 hour at room temperature, and then added to cultured HepG2-NTCP cells, and the cells were cultured for 7 days while changing the medium with PMM medium containing 2.5% DMSO every 2 hours. Intracellular HBV DNA was extracted from the infected HepG2-NTCP cells and subjected to Southern blot hybridization.

As a result, it was confirmed that the AbS0 antibody exhibited a HBV neutralizing activity in a dose-dependent manner, whereas mouse IgG did not (FIG. 4B).

Through this, it has been confirmed that AbS0 has the ability to neutralize HBV infection.

The above results suggest that AbS0, the novel antibody of the present invention, can bind to all HBV genotypes A to D and neutralize them, and suggest that the novel AbS0 antibody is effective in preventing infection with the virus genotypes or treating patients with hepatitis caused by viral infection. In addition, this suggests that the infection with HBV genotypes A to D can also be specifically detected.

The light chain variable region of the novel antibody AbS0 of the present invention was represented by SEQ ID NO: 14, and the heavy chain variable region was represented by SEQ ID NO: 15.

### Example 5: Determination of fine epitope of AbS0 antibody

In order to understand the mechanism of the HBV neutralizing activity of AbS0 antibody, the binding site (fine epitope) in preS1 of AbS0 was precisely determined and analysis was performed to examine whether the amino acid sequence of this fine epitope is conserved among the genotypes of HBV.

### 5-1 Alanine scanning mutagenesis of PreS1(19-34)

Since the AbS0 antibody was discovered through panning of a human antibody library using the preS1(20-32) peptide of genotype C, in order to determine which residues within the preS1 (20-32) region bind to the AbS0 antibody, mutants in which each residue of preS1 (19-34) of genotype C was substituted to alanine were constructed, and the antigen binding activity of the AbS0 antibody to each of the mutants was analyzed.

Specifically, DNA was synthesized in which each residue of preS1 (19-34) was substituted to alanine by a recombinant PCR method using primers containing a mutant sequence, digested with *BamHI* and *EcoRI* (NEB) restriction enzymes, and then subcloned into the *BamHI-EcoRI* sites of the pGST-preS1(1-56)-strep expression plasmid, containing the wild type preS1 sequence, to construct an expression plasmid carrying an alanine-substitution mutant instead of the wild type preS1(1-56). Afterwards, in order to express each mutant, recombinant *E. coli* DH5α was cultured in a medium containing 0.2 mM IPTG at 18°C for 19 hours. After the cells were disrupted by sonication, the GST-preS1(1-56)-strep protein was purified by affinity chromatography using glutathione beads (Genscript), and the purity of the purified protein was analyzed by 12% SDS-PAGE (FIG. 5).

### 5-2. Analysis of antigen binding activity of antibody to alanine-substitution mutant of preS1

The antigen binding activity of the AbS0 antibody to each alanine-substitution mutant was analyzed by indirect ELISA (FIG. 6).

Specifically, each well was coated with the mutated GST-preS1(1-56)-strep antigen (200 ng) and then incubated with the serially diluted AbS0 antibody. The bound AbS0 antibody was detected using anti-human IgG Fc-HRP (1:10,000 v/v, Thermo) as a secondary antibody.

As a result, it was confirmed that the L22A, G23A or F25A mutant completely lost antibody binding activity, whereas the other mutants showed the same activities as the wild type antigen. This result indicates that the AbS0 antibody directly binds to Leu22, Gly23, and Phe25 of the preS1 antigen.

### Example 6: Determination of epitope binding site (paratope) of AbS0 antibody and identification of mutant with increased affinity

In order to identify the amino acid residues of the AbS0 antibody that directly bind to the antigen, the CDR residues were subjected to alanine scanning mutagenesis and site-directed mutagenesis, and then the antigen binding activities of these mutants were analyzed.

### 6-1. Alanine scanning mutagenesis of HCDR3 and LCDR3 of AbS0 antibody

In order to construct mutants in which each residue of HCDR3 (V95, I96, Y97, S99, and L100 based on SEQ ID NO: 15) and LCDR3 (S91, Y92, S93, S94, and Y96 based on SEQ ID NO: 14) of the AbS0 antibody was substituted to alanine, mutated VH or VL gene was synthesized by a recombinant PCR method using primers containing a mutated residue (Table 2). In the case of HCDR3 mutants, each synthesized VH gene was digested with *EcoRl* and *Apal* restriction enzymes and then subcloned into the *EcoRl-Apal* site of the expression vector (pCMV-dhfr-AbS0H) containing the heavy chain gene of the AbS0 antibody. In the case of LCDR3 mutants, each synthesized VL gene was digested with *HindIII* and *BsiWl* restriction enzymes, and then subcloned into the *HindIII-BsiWl* site of the expression vector (pCMV-dhfr-AbS0k) containing the light chain gene of the AbS0 antibody. Expression plasmids of a total of 10 alanine-substitution mutants were constructed as follows.

**[Table 2]**

| | | | |
|---|---|---|---|
| L3-v1 | S91A | H3-v1 | V95A |
| L3-v2 | Y92A | H3-v2 | I96A |
| L3-v3 | S93A | H3-v3 | Y97A |
| L3-v4 | S94A | H3-v4 | S99A |
| L3-v5 | Y96A | H3-v5 | L100A |

In order to produce each mutant antibody, the constructed expression plasmids were mixed with polyethyleneimine (PEI) at a ratio of 1:4 (30 µg : 120 µg) and then introduced into HEK293F cells. The transformed cells were cultured for 7 days to express mutant antibodies, then the culture supernatants were obtained, and the antigen binding activities of these were analyzed by indirect ELISA. Specifically, each well was coated with the recombinant GST-preS1(1-56)-strep protein (100 ng) of HBV genotype C, then the serially diluted AbS0 antibody or AbS0 mutant was added, and anti-human IgG Fc-HRP (1:10,000 v/v, Thermo) was added as a secondary antibody for reaction.

The concentration of AbS0 antibody or AbS0 mutant used in indirect ELISA was determined by quantitative ELISA. Each well was coated with anti-human IgG kappa antibody (100 ng, Thermo), then the serially diluted AbS0 antibody or mutant was added, and anti-human IgG Fc-HRP (1:10,000 v/v, Thermo) was added as a secondary antibody for reaction.

As a result, HCDR3 mutant L100A and LCDR3 mutant Y96A exhibited no or very low antigen binding activity, but the other six mutants (V95A and S99A of HCDR3; S91A, Y92A, S93A, and S94A of LCDR3) exhibited the same antigen binding activities as the wild type AbS0 (FIG. 7). The CDR3 mutants I96A and Y97A were not expressed and therefore exempted from the analysis. From these results, it has been confirmed that Leu100 of HCDR3 and Tyr96 of LCDR3 of AbS0 are critical in antigen binding.

### 6-2 Site-directed mutagenesis of HCDR1 and HCDR2 of AbS0 antibody

In general, in antibodies, amino acid position 33 in the HCDR1 and position 50 in the HCDR2 vary from antibody to antibody, and thus are known to be involved in antigen binding. In the case of AbS0 antibody, site-directed mutagenesis was performed to analyze whether residues 33 and 50 are involved in antigen binding. (Positions 33 and 50 are positions based on SEQ ID NO: 15.)

In the case of AbS0 antibody, since position 33 in the HCDR1 is alanine, alanine was substituted to eight other residues (S, P, V, T, G, D, L, and N) in order to investigate whether this residue is involved in antigen binding, and serine at position 50 (Ser50) in the HCDR2 was substituted to six other residues (A, Y, E, F, V, and P). In order to construct the mutant genes, recombinant PCR was performed using degenerate primers. The synthesized VH genes were digested with *EcoRl* and *Apal* restriction enzymes and then subcloned into the *EcoRl-Apal* site of pdCMV-dhfr-AbS0 to obtain expression plasmids for the mutants. It was confirmed that a total of 14 mutants described above were secured through sequencing. Each expression plasmid was introduced into HEK293F cells, and the culture supernatant obtained after culture for 7 days was analyzed by indirect ELISA and quantitative ELISA, as described in Example 6-1.

As a result, in the case of position 33 in the HCDR1, when alanine was substituted to asparagine (A33N, FIG. 8A), leucine (A33L, FIG. 8B), or aspartic acid (A33D, FIG. 8C), the mutants did not exhibit any antigen binding activities, and the glycine-substituted mutant (A33G, FIG. 8C) also exhibited significantly decreased antigen binding activity compared to that of the wild type AbS0 antibody. This result indicates that Ala33 is involved in antigen binding. On the other hand, the three mutants A33S (FIG. 8A), A33V, and A33P (FIG. 8B) exhibited the antigen binding activities, similar to that of the wild type antibody.

In the case of the mutants of Ser50 of HCDR2, it was confirmed that the S50A mutant exhibited a slightly increased antigen binding activity compared to that of the AbS0 antibody, whereas antigen binding activity of S50Y mutant was greatly decreased and those of the S50F and S50E mutants were completely lost (FIG. 8D). In the case of S50V and S50P mutants, expression was not detected and therefore antigen binding activity was not analyzed. The results indicate that position 50 in the HCDR2 is important for antigen binding and that Ala50 is more optimal for antigen binding than Ser50.

In order to analyze whether Gln52a (position 4 based on SEQ ID NO: 11) of HCDR2 of the AbS0 antibody is also involved in antigen binding, mutants in which glutamine was substituted to four other amino acids (Y, A, F, and M) were constructed and transiently expressed in HEK293F cells. As a result, the mutants (Q52aA, A52aF, and A52aM) exhibited the same antigen binding activities as the AbS0 antibody, and the antigen binding activity of Q52aY mutant was not analyzed since the expression thereof was not detected. These results suggest that Gln52a of HCDR2 is not involved in the interaction with the preS1 antigen.

Based on the results of Examples 5 and 6, it has been concluded that in the AbS0 antibody, Ala33 of HCDR1, Ser50 of HCDR2, Leu100 of HCDR3, and Tyr96 of LCDR3 directly bind to Leu22, Gly23, and Phe25 of preS1. In addition, through the experiment of Example 6, mutant S50A having improved antigen binding activity compared to AbS0 was obtained, and this mutant antibody was named "AbS1".

### Example 7: Analysis of antigen binding properties of AbS1 antibody

### 7-1 Comparison of antigen-binding activities between AbS1 and AbS0 to preS1 antigen of genotypes A and C to preS1 antigen of genotypes A and C

In Example 6-2, the recombinant GST-preS1(1-56)-strep antigen of HBV genotype C was used to analyze the antigen binding ability of the AbS0 antibody and mutants, and indirect ELISA was performed on the recombinant GST-preS1(1-56)-strep antigen of genotype A to analyze whether the AbS1 antibody exhibits higher binding activity than AbS0 to the preS1 antigen of other genotypes. As a result, it was confirmed that AbS1 exhibited higher antigen binding activity to the preS1 of genotype A than AbS0 (FIG. 9).

### 7-2 Analysis of binding activity of AbS1 antibody to the preS1 antigen having different receptor binding motif sequence

Since the epitope (Leu22, Gly23, and Phe25 of preS1) of the AbS1 antibody is located within the HBV receptor binding motif, the sequences of the receptor binding motifs (aa 20-26, genotype A) of 9639 (as of April 30, 2021) preS1 genes registered in the HBV database (https://hbvdb.lyon.inserm.fr/HBVdb/) were analyzed to estimate whether the AbS1 antibody can broadly bind to HBV existing on Earth. As a result, 96.6% of preS1 sequences had the NPLGFFP sequence as a receptor binding motif, and 2.6% had NPLGFLP containing leucine (Leu25) instead of phenylalanine (Phe25) at position 25 (Table 3).

**[Table 3]**

| Proportion of receptor binding motif having NPLGFFP or NPLGFLP in HBV genotypes A to H | | | | | | | | |
|---|---|---|---|---|---|---|---|---|
| **Receptor binding motif (number)** | **Genotype** | | | | | | | |
| | **A** | **B** | **C** | **D** | **E** | **F** | **G** | **H** |
| NPLGFF P (9312) | 1355 (96.50%) | 2751 (98.18%) | 3055 (97.73%) | 1453 (99.18%) | 432 (96.86%) | 234 (81.53%) | 1 (1.28%) | 31 (100%) |
| NPLGFL P(247) | 36 (2.56%) | 30 (1.07%) | 38 (1.22%) | 8 (0.55%) | 5 (1.12%) | 53 (12.47%) | 77 (98.72%) | 0 (0.00%) |
| Others (80) | 13(9 D.S.) (0.93%) | 21 (12 D.S.) (0.75%) | 33(18 D.S.) (1.06%) | 4(3 D.S.) (0.27%) | 9(8 D.S.) (2.02%) | 0 (0.00%) | 0 (0.00%) | 0 (0.00%) |
| Total (9639) | 1404 | 2802 | 3126 | 1465 | 446 | 287 | 78 | 31 |

| | | | | | | | | |
|---|---|---|---|---|---|---|---|---|
| ***different sequences** | | | | | | | | |

Therefore, in order to analyze whether the AbS1 antibody also binds to NPLGFLP, the GST-preS1(1-56)-strep antigen of genotype A having the NPLGFLP sequence was expressed and purified and indirect ELISA was performed. As a result, it was confirmed that the AbS1 antibody binds well to NPLGFLP, although the binding activity is slightly lower than that to NPLGFFP (FIG. 9).

From the above results, it has been confirmed that the AbS1 antibody exhibits higher antigen binding activity than AbS0 to the preS1 antigen of genotypes A and C, and it is predicted that both antibodies can bind well to HBV, which has a hepatocyte receptor binding motif of the NPLGFLP sequence.

### 7-3 Analysis of binding activity of AbS1 antibody to preS1 antigen of genotypes E and F

Through Examples above, it was confirmed that the AbS1 antibody can bind to the preS1 antigen of HBV genotypes A to D and the mutant . Therefore, to examine whether the AbS1 antibody can also bind to the preS1 antigen of genotypes E and F, quantitative ELISA (FIG. 10A) and indirect ELISA (FIG. 10B) were performed using the recombinant GST-preS1(1-56)-strep antigen of genotypes E and F (Fig. 1). At this time, the F25A mutant preS1 antigen of genotype C in Example 5 was used as a negative control.

As a result, it was confirmed that AbS1 binds well to the preS1 of genotypes E and F (FIG. 10).

Taken together, the above results indicate that the novel antibodies of the present invention, AbS0 and AbS1, can bind to all HBV genotypes A to F and bind to the hepatocyte receptor binding motif of preS1, thus have the mechanism of action of an HBV entry inhibitor that not only neutralizes HBV but also effectively inhibits HBV infection by blocking HBV from entering hepatocytes, and can be usefully used for the prevention and treatment of HBV infection.

From the above description, those skilled in the art to which the present invention pertains will understand that the present invention can be implemented in other specific forms without changing its technical idea or essential features. Therefore, it should be understood that the embodiments are not limitative, but illustrative in all aspects. The scope of the present invention is defined by the appended claims rather than by the description preceding them, and therefore all changes and modifications that fall within metes and bounds of the claims or equivalents of such metes and bounds are intended to be embraced by the claims.

## Claims

1. An antibody that binds to an amino acid at a specific position in a preS1 antigen of HBV (hepatitis B virus), wherein
the specific position in the preS1 antigen corresponds to positions 22, 23, and 25 based on SEQ ID NO: 19.

2. The antibody according to claim 1, wherein the antibody has tyrosine at position 8 in an LCDR3 sequence of SEQ ID NO: 5 and leucine at position 6 in an HCDR3 sequence of SEQ ID NO: 9 as paratopes.

3. The antibody according to claim 1, wherein the antibody has amino acid at position 1 in an HCDR2 sequence represented by SEQ ID NO: 8 as a paratope.

4. The antibody according to claim 3, wherein the amino acid residue at position 1 in the HCDR2 sequence represented by SEQ ID NO: 8 is alanine or serine.

5. The antibody according to claim 1, comprising:
(a) a light chain variable region including a light chain CDR1 of SEQ ID NO: 3, a light chain CDR2 of SEQ ID NO: 4, and a light chain CDR3 of SEQ ID NO: 5; and
(b) a heavy chain variable region including a heavy chain CDR1 of SEQ ID NO: 7, a heavy chain CDR2 of SEQ ID NO: 8, and a heavy chain CDR3 of SEQ ID NO: 9.

6. The antibody according to claim 1, comprising:
(a) a light chain variable region including a light chain CDR1 of SEQ ID NO: 3, a light chain CDR2 of SEQ ID NO: 4, and a light chain CDR3 of SEQ ID NO: 6; and
(b) a heavy chain variable region including a heavy chain CDR1 of SEQ ID NO: 10, a heavy chain CDR2 of SEQ ID NO: 11 or 12, and a heavy chain CDR3 of SEQ ID NO: 13.

7. The antibody according to claim 1, wherein the antibody specifically binds to one or more genotypes selected from HBV genotype A, B, C, D, E, or F.

8. The antibody according to claim 1, wherein the light chain variable region of the antibody consists of SEQ ID NO: 1.

9. The antibody according to claim 1, wherein the heavy chain variable region of the antibody consists of SEQ ID NO: 2.

10. The antibody according to claim 1, comprising a light chain variable region represented by SEQ ID NO: 14; and a heavy chain variable region represented by SEQ ID NO: 15 or 16.

11. A polynucleotide encoding the antibody according to any one of claims 1 to 10.

12. An expression vector comprising the polynucleotide according to claim 11.

13. A host cell comprising the expression vector according to claim 12.

14. A pharmaceutical composition for prevention or treatment of HBV infection, comprising the antibody according to any one of claims 1 to 10.

15. The composition according to claim 14, wherein the composition is for prevention or treatment of hepatitis B.

16. A method for diagnosing HBV infection, the method comprising detecting a preS1 protein present in a biological sample isolated from a subject suspected of being infected with HBV through an antigen-antibody reaction using the antibody according to any one of claims 1 to 10.

17. The method according to claim 12, wherein the method is for diagnosis of hepatitis B.

18. A composition for detection of HBV, comprising the antibody according to any one of claims 1 to 10.

19. The composition according to claim 18, wherein the composition is for diagnosis of hepatitis B.

20. A kit for detection of HBV, comprising the composition according to claim 18.
